# EUROPEAN PATENT SPECIFICATION

(11) **EP 1 007 514 B1**
(45) Date of publication and mention of the grant of the patent: **15.11.2006**
(21) Application number: 98940992.5
(22) Date of filing: 19.08.1998
(51) Int. Cl.: C07D 235/12, A61K 31/47, C07D 277/64, C07D 233/54, C07D 215/14

(54) **HETEROCYCLIC KETONES AS NPY Y5 ANTAGONISTS**
HETEROZYCLISCHE KETONEN ALS NPY Y5 ANTAGONISTEN
CETONES HETEROCYCLIQUES EN TANT QU'ANTAGONISTES DU RECEPTEUR Y5 DU NEUROPEPTIDE Y

(30) Priority: 25.08.1997 US 920187
(43) Date of publication of application: 14.06.2000
(73) Proprietor: BAYER CORPORATION, Pittsburgh, PA 15205 (US)
(72) Inventor: CONNELL, Richard, D., Trumbull, CT 06611 (US); HERTZOG, Donald, L., Durham, NC 27707 (US); BRINI, Warren, Branford, CT 06405 (US); CAMPBELL, Ann-Marie, Monroe, CT 06468 (US); GUNN, David, E., Hamden, CT 06517 (US); PELLETIER, Roberta, L., Woodbury, CT 06798 (US)
(74) Representative: Burkert, Frank
(86) International application number: PCT/US1998/017164
(87) International publication number: WO 1999/010330

(56) References cited:
- WO-A-97/19682
- WO-A-97/20823
- WALTER RIED ET AL.: "Synthese und Reaktivität von 4-Arylsulfonyl-2-hydroxy-3-phenyl-2-cyclob uten-1-onen und 4-Arylsulfonyl-3-phenyl-3-cyclobuten-1,2-d ionen" CHEMISCHE BERICHTE., vol. 108, - 1975 pages 538-553, XP002082865 WEINHEIM DE

## Description

### BACKGROUND OF THE INVENTION

### FIELD OF THE INVENTION

This invention relates to novel heterocyclic ketone compounds pharmaceutical compositions and methods of treating Neuropeptide Y (NPY) Y5 receptor mediated conditions with such compositions.

### SUMMARY OF RELATED ART

NPY is a 36-amino acid peptide neurotransmitter that is located throughout the central and peripheral nervous systems. (Tatemoto, *Proc. Natl. Acad. Sci.* USA **79:** 5485 (1982); Hazlewood, *Proc. Soc. Exp. Biol. Med.,* **202:44** (1993).) It affects a broad range of phenomena, including blood pressure regulation, memory, anxiolysis/sedation, food and water appetite, vascular and other smooth muscle activity, intestinal electrolyte secretion, and urinary sodium excretion. (*E.g.,* Comers and Wahlestedt, *The Biology of Neuropeptide Y and Related Peptides,* Humana Press, Totowa, NJ, (1993); Kalra et al., *Phys, & Behavior,* **50**:5 (1991).)

At least five NPY receptor subtypes have been identified: Y1, Y2, Y3, Y4/PP and Y5. Affinity for NPY, as well as peptide YY, and various fragments thereof, varies among the receptor subtypes. While other NPY receptor subtypes are believed to mediate such conditions as blood pressure, it is believed that the NPY Y5 receptor is linked to such abnormal conditions as obesity, bulimia nervosa, sexual/reproductive disorder, depression, anxiety, gastric ulcer, memory loss, migraine, pain, epileptic seizure, hypertension, cerebral hemorrhage, shock, congestive heart failure, sleep disturbance, nasal congestion, and diarrhea. (US Patent No. 5,602,024, Feb. 11, 1997).

WO 9720823 and WO 9719682 disclose some NPY S antagonists, Chem. Ber. 108, 538 (1975) discloses the preparation propanone derivatives substituted by heterocyclic rings.

Therefore, there is a need for compounds that selectively inhibit the NPY Y5 receptor and act specifically as effective Y5 receptor antagonists for treating-Y5 receptor mediated conditions.

### DESCRIPTION OF THE INVENTION

This invention specifically relates to heterocyclic ketone compounds of the general formula:
wherein
- R: is C₁-C₆-alkyl, C₁-C₆-alkoxy, F, Br, Cl and I;
- Ar¹: is phenyl, naphthyl or thiophene, which can optionally be substituted
with R;
- Ar²: is phenyl or pyridyl, which can optionally be substituted with R;
- n: is 0, 1 or 2;
- Het: is quinoline, benzimidazole, benzthiazole, indole, indoline, purine, imidazole, pyrroline, pyrrolidine, pyrazole, pyrazoline or triazole, where the heteroatom groups of said moiety are in the α position of the carbon atom bound to the carbonyl atom, and where in one of the position alpha of the carbon bound to the carbonyl there is N atom
which can optionally be substituted with zero to six substituents as defined by R;
and pharmaceutically acceptable salts thereof, with the exception of the following specific compounds: 1-(2-benzothiazolyl)-3-[(4-methylphenyl) sulfonyl]-2-phenyl-1-propanone, 1-(1-methyl-1H-benzimidazole-2-yl)-3-[(4-methylphenyl)sulfonyl]-2-phenyl-1-propanone, 1-(1-H-benzimidazole-2-yl)-3-(4-[(methylphenyl)sulfonyl]-2phenyl-1-propanone and 1-(1H-benzimidazole-2-yl)-3-[phenyl(sulfonyl)]-2phenyl-1 propanone.

C₁-C₆ alkyl means straight or branched chain alkyl groups having from about one to about six carbon atoms and includes such groups as methyl, ethyl, n-propyl, isopropyl, n-butyl, isobutyl, sec-butyl, tert-butyl, as well as vinyl, allyl, propynyl, butenyl, butadienyl, isopropenyl, and the like. The term C₁-C₆ alkoxy means straight or branched chain alkoxy groups having from about one to about six carbon atoms, and includes such groups as methoxy, ethoxy, n-propoxy, isopropoxy, n-butoxy, isobutoxy, sec-butoxy, tert-butoxy, and the like.

Ar¹ can be optionally substituted with zero to six R substituents which can be located at any available position on the Ar¹ ring, and, when there are 2 or more R substituents, can be the same or different.

When Ar² is optionally substituted, it can be substituted with zero to five R substituents that can be located at any available position on the Ar² moiety, and that, when there are 2 or more R substituents, can be the same or different.

Illustrative examples of the compounds of this invention include the following compounds of Formula I:

**Table I**

| Compound Number | Compound Name |
|---|---|
| 1 | (4-Methylphenylsulfonyl)-1-oxo-2-phenyl-1-(quinolin-2-yl)propane |
| 2 | (1*H*-Imidazol-2-yl)-2-phenyl-3-p-tolylsulfanyl-propan-1-one |
| 3 | (1*H*-Imidazol-2-yl)-2-phenyl-3-(toluene-4-sulfonyl)-propan-1-one |
| 4 | (1*H*-Benzoimidazol-2-yl)-2-phenyl-3-p-tolylsulfanyl-propan-1-one |
| 5 | (1*H*-Benzoimidazol-2-yl)-2-phenyl-3-(toluene-4-sulfinyl)-propan-1-one |
| 6 | 1-Benzothizol-2-yl-2-phenyl-3-(toluene-4-sulfonyl)-propan-1-one |
| 7 | (5,6-Dimethyl-1*H*-benzoimidazol-2-yl)-2-phenyl-3-(toluene-4-sulfonyl)-propan-1-one |
| 8 | 3-Benzenesulfonyl-1-(1*H*-benzoimidazol-2-yl)-2-phenyl-propan-1-one |
| 9 | (1*H*-Benzoimidazol-2-yl)-2-(4-fluoro-phenyl)-3-(toluene-4-sulfonyl)-propan-1-one |
| 10 | (1*H*-Benzoimidazol-2-yl)-3-(4-methoxy-benzenesulfonyl)-2-phenyl-propan-1-one |
| 11 | (1*H*-Benzoimidazol-2-yl)-3-(4-bromo-benzenesulfonyl)-2-phenyl-propan-1-one |
| 12 | (1*H*-Benzoimidazol-2-yl)-3-(naphthalene-1-sulfonyl)-2-phenyl-propan-1-one |
| 13 | (5-Methyl-1*H*-benzoimidazol-2-yl)-2-phenyl-3-(toluene-4-sulfonyl)-propan-1-one |
| 14 | (5-Methoxy-1*H*-benzoimidazol-2-yl)-2-phenyl-3-(toluene-4-sulfonyl)-propan-1-one |
| 15 | (1*H*-Benzoimidazol-2-yl)-2-phenyl-3-(toluene-4-sulfonyl)-propan-1-one |

As is true of most classes of therapeutically effective compounds, certain subclasses and certain species which are especially effective are preferred over others. In this instance, those compounds of Formula I which are preferred include those compounds where the heterocyclic moiety is benzimidazole or substituted benzimidazole, and both Ar¹ and Ar² are phenyl or substituted phenyl. Most preferred compounds include Compound 8 and Compound 13.

Representative salts of the compound of figure I are those salts formed with non-toxic organic or inorganic acids, such as, for example, those formed from the following acids: hydrochloric, hydrobromic, sulfuric, phosphoric, nitric, maleic, fumaric, benzoic, ascorbic, succinic, methanesulfonic, acetic, propionic, tartiaric, citric, lactic, malic, mandelic, cinnamic, palmitic, itaconic and benzenesulfonic.

The particular process to be utilized in the preparation of the compounds of this invention depends upon the specific compound desired. Such factors as the selection of the specific aryl moieties, the specific heterocyclic moiety, the presence or absence of one or two oxygen atoms in conjunction with the sulfur atom, and the specific substituents on the aryl and heterocyclic moieties all play a role in the path to be followed in the preparation of the specific compounds of this invention. Those factors are readily recognized by one of ordinary skill in the art. However, in general, the compounds of this invention in which X is NH and n is 2 may be prepared by the process described in Chem. Ber., *Reactions with Cyclobutenediones,* XXXV, **108**:538-553 (1975) (the "Reid process"), which is incorporated herein by reference, or by standard techniques and known processes analogous thereto. Generally, that process is illustrated by the following Reaction Scheme 1: 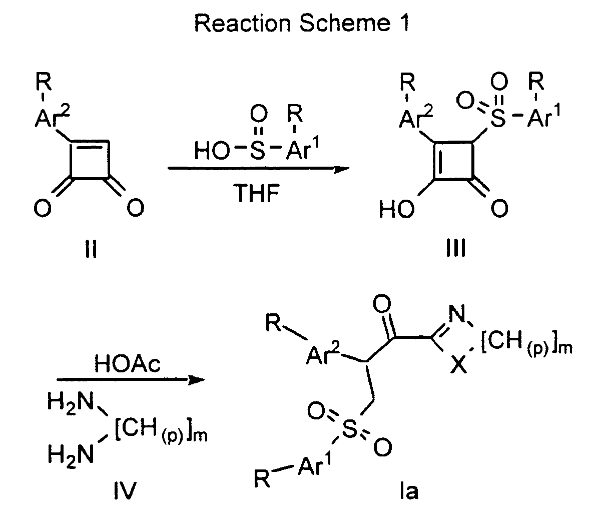 The cyclobutenedione of Formula II is coupled with an aryl sulfonic acid in a solvent such as tetrahydrofuran (THF) under reflux conditions to produce the sulfonated compound of Formula III, which in turn is converted to the appropriate end product (Formula la) by reacting it with the appropriate diamine compound of Formula IV in the presence of a solvent such as acetic acid (HOAc). The diamines of formula IV are employed generally in an amount of from about 0.5 to 3.0 mol, preferably from 1 to 2 mols, relative to 1 mol of cyclobutanedione of Formula III. The process is generally carried out at normal pressure and at a temperature of about 10°C to about 60°C.

In general, the compounds of this invention in which X is S or CH and n is 2 may be prepared by either of two methods. The first process is described in the patent DE 3527334 A1, which is incorporated herein by reference, or would be operable by standard techniques and known processes analogous thereto. Generally, that process is illustrated by the following Reaction Scheme 2:

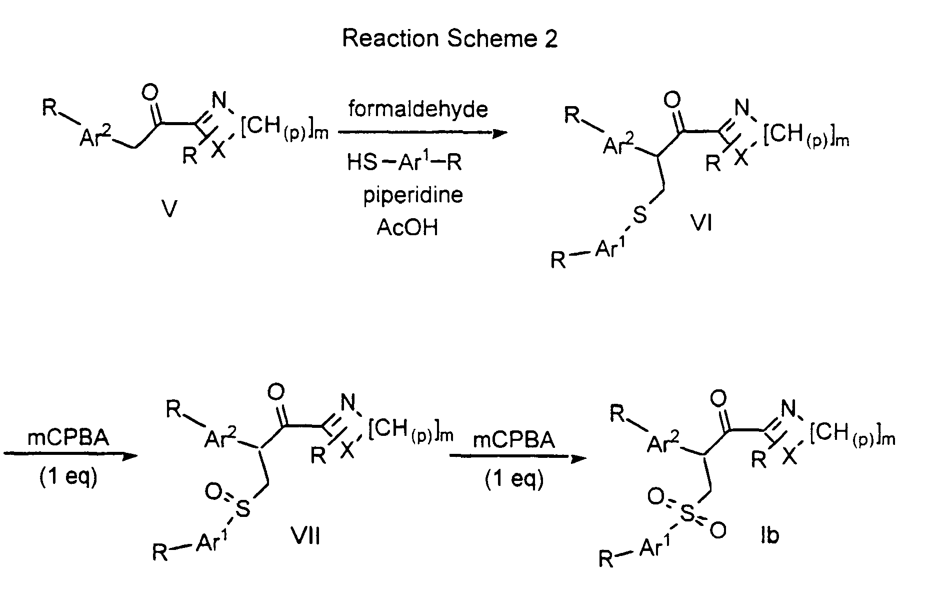

Heterocyclic ketones of Formula V are employed generally in an amount of about 1 mol relative to about 1 mol of formaldehyde or formaldehyde equivalent, about 1 mol of aryl thiol, about 0.1-1.0 mol of acetic acid, and 0.1 to 1.0 mol of piperidine in a solvent such as toluene at about 50 - 150°C, preferably 100-125°C, to produce the compound of Formula VI. The compound of Formula VI is then in turn converted to the sulfide of Formula VII with about one equivalent of 3-chloroperoxybenzoic acid (mCPBA) in a solvent such as dichloromethane. The sulfide of Formula VII is then converted to the sulfone end product (Formula 1b) by reacting it with about one or more equivalents of mCPBA in a solvent such as dichloromethane. Alternatively, the sulfide of Formula VI may be directly transformed to the sulfone end product (Formula 1b) by treatment with two equivalents or greater of mCPBA.

This process (Reaction Scheme 2) may also be used to prepare compounds of this invention in which X is S and n is 0 or 1 (Formulae VI and VII, respectively).

The second method for preparing compounds of this invention in which X is CH or S is described in *Chem. Ber.* **1965,** *98,* 638 (the Hellmann process), which is incorporated herein by reference, or by standard techniques and known processes analogous thereto. Generally, that process is illustrated by the following Reaction Scheme 3:

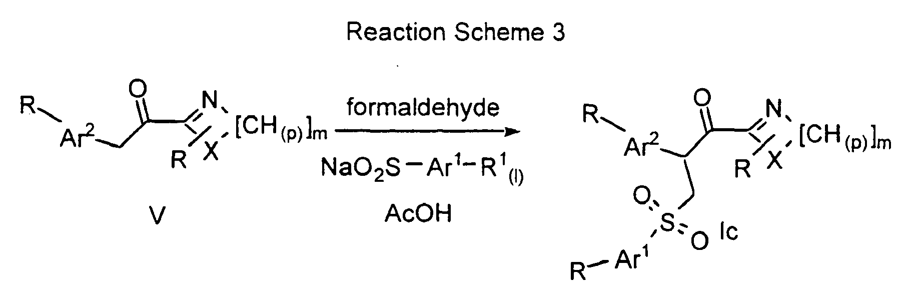

The ketones of Formula V are employed generally in an amount of about 1 mol relative to about 1 to 2 mols of formaldehyde or formaldehyde equivalents, about 1 to 3 mols of sodium aryl sulfite, and about 1 to 3 mols of acetic acid (AcOH) in a solvent such as DMF at about 45 - 90°C to produce the compounds of Formula 1c.

It has been discovered that the compounds of this invention in which X is NH and n is 0, 1, or 2 may be prepared by a first novel process which is illustrated by the following general Reaction Scheme 4:

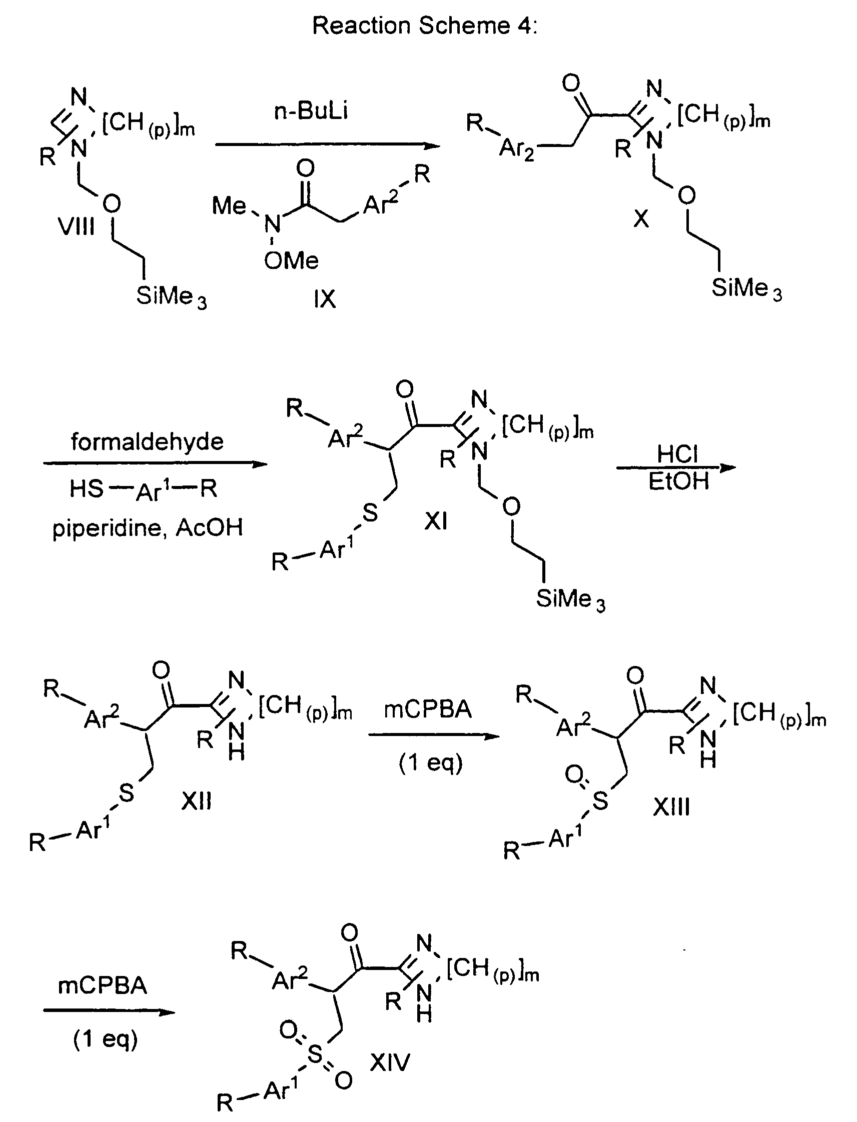

The heterocycle of Formula VIII, where SEM is a protecting group as defined below, is employed in an amount of about 1 mol relative to 1 mol of an organolithium such as n-butyllithium in a solvent such as THF at about -78°C. Following treatment of the compound of Formula VIII with an organolithium such as n-butyl lithium, the amide of Formula IX is added in an amount of about 1 mol relative to 1 mol of heterocycle of Formula VIII, to provide the ketone of Formula X. This compound, in turn, is employed generally in an amount of about 1 mol relative to about 1 mol of formaldehyde for formaldehyde equivalent, about 1 mol of aryl thiol, about 0.1 to 1.0 mol of a carboxylic acid such as acetic acid, and about 0.1 to 1.0 mol of a dialkylamine such as piperidine in a solvent, preferably toluene, at about 50 - 150°C, preferably 100 - 125 °C to provide the sulfide of Formula XI. The compound of Formula XI is then stirred at about 30 - 100°C, preferably at about 60-70°C, in about a 1:1 mixture of ethanol:concentrated hydrochloric acid to remove the 2 trimethylsilanyl-ethoxymethyl (SEM) protecting group and provide the sulfide of Formula XII. The sulfide XII is then converted to the sulfoxide XIII through the use of about one equivalent of an oxidizing agent, preferably mCPBA, in a solvent such as dichloromethane, or by other means well known in the art. The sulfoxide of Formula XIII is then converted to the sulfone of Formula XIV by reacting it with one or more equivalents of an oxidizing agent, preferably mCPBA, in a solvent such as dichloromethane, or by other means well known in the art. The sulfide of Formula XII also may be directly transformed to sulfone XIV by treatment with about two equivalents or greater of an oxidizing agent such as mCBPA.

This process makes possible the synthesis of sulfoxides of Formula XIII, which cannot be synthesized from the known procedures of Reid or Hellmann described above. In this first process of this invention, it is critical to remove the protecting group from the compound of Formula XI to make the sulfide of XII, which, in turn, can be oxidized by standard methods known in the art to form the sulfoxides and sulfones of Formulae XIII and XIV. Removal of the protecting group at the indicated stage (from sulfides XI) is critical as attempted removal from the corresponding sulfoxides or sulfones leads to decomposition.

The protecting groups used to protect NH containing heterocycles are standard protecting groups that are described in the literature (*e.g.,* Greene, T.W. et. al. *Protective Groups in Organic Synthesis,* 1991, John Wiley & Sons). These include sulfonyl, carbamates, acyl, benzoyl, alkyl, benzyl, silyl, hemiacetal, and the like, groups. The removal of these protecting groups are also described in the same literature reference or are otherwise known by one skilled in the art. The use of the [2-(trimethylsilyl)ethoxy]methyl (SEM) protecting group and its deprotection with HCl is preferred.

The formaldehyde equivalents, such as paraformaldehyde and s-trioxane, are in general employed in an amount from about 0.5 to 3 mol, preferably from 1 to 1.5 mol, relative to 1 mol of the corresponding ketones. In general, the nucleophiles S-Ar¹-R are employed in an amount from 0.05 to 3.0 mol, preferably from 1 to 2 mol, relative to 1 mol of the ketone compound.

A second novel process comprising an improvement over the known process described above for making the compounds of this invention in which X is NH and n is 2 is in the use of a catalyst in Reaction Scheme 1, as depicted in Reaction Scheme 5: 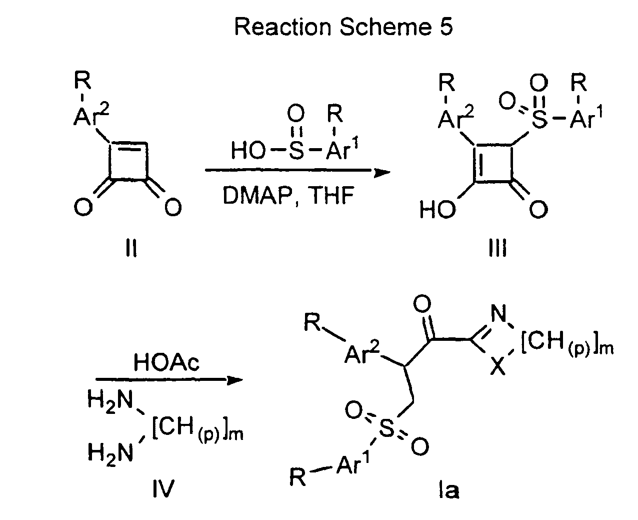 The novelty of this process is in the use of a catalytic amount of any alkyl amine (DMAP, in the scheme above), preferably a tri- or dialkylamine compound, most preferably 4-dialkylaminopyridine, in the transformation of intermediate II to intermediate III. Catalysts that are effective in this improved process step include amines such as 4-dimethylaminopyridine, 4-pyrrolidinopyridine, triethylamine, and diisopropylethylamine. The use of such a catalyst provides significantly higher yields in this reaction than does the uncatalyzed process described as the Reid process above. This process improvement demonstrates about a 79% yield compared to about a 20% yield attained from the Reid process. Suitable solvents for the process (THF. in the scheme above) are customary organic solvents which do not change under the reaction conditions. These preferably include ethers such as diethyl ether, dioxane, tetrahydrofuran, glycol dimethyl ether, or alcohols, for example methanol, ethanol, propanol, isopropanol, butanol, isobutanol or tert-butanol, or hydrocarbons such as benzene, toluene, xylene, hexane, cyclohexane or petroleum fractions, or halogenated hydrocarbons such as dichloromethane, trichloromethane, tetrachloromethane, dichloroethylene, trichloroethylene, chlorobenzene, fluorobenzene, or benzotrifluoride, or ethyl acetate, triethylamine, pyridine, dimethylsulphoxide, dimethylformamide, hexamethylphosphoramide, acetonitrile, acetone, nitromethane, formic acid, acetic acid, or propanoic acid. It is also possible to use mixtures of the solvents mentioned. Tetrahydrofuran is preferred.

This process is in general carried out in a temperature range of from about - 90°C to about +150°C, preferably from about 0°C to about +120°C. Furthermore this is in general carried out at normal pressure. However, it is also possible to carry out the processes at elevated pressure or at reduced pressure (e.g. in a range from 0.5 to 5 bar).

The foregoing reaction schemes are further illustrated by the specific examples described later herein.

Certain intermediate compounds useful in the manufacture of the pharmaceutical compounds of this invention are novel. 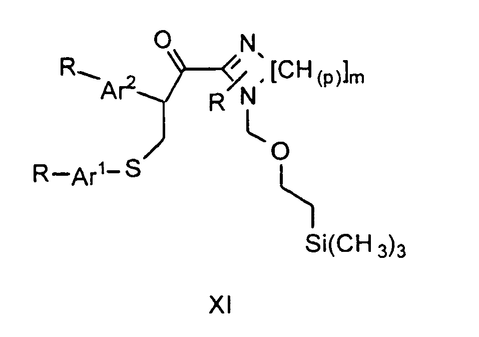 wherein
R is C₁-C₆ alkyl, C₁-C₆ alkoxy, F, Br, Cl, and I
Ar¹ is phenyl, naphthyl or thiophene;
Ar² is phenyl or pyridyl;
X is CH₂, NH or S;
m is 2 through 7; and
p is 0, 1, or 2;
and 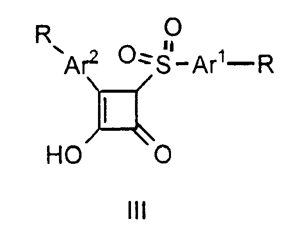 wherein
R is C₁-C₆ alkyl, C₁-C₆ alkoxy, F, Br, Cl, and I
Ar¹ is phenyl or naphthyl; and
Ar² is phenyl.
The variables, such as the R groups, Ar groups, and the like, in the intermediates of Formulae XI and III above are the same as those described for compounds of the Formula I above.

It is readily apparent that the intermediates of Formulae XI and III can be prepared by the general reaction schemes described above, and are further illustrated by the specific examples described later herein. Illustrative examples of the novel intermediate compounds of this invention are noted in the specific examples below as compounds designated as Novel Intermediates.

The end product compounds of this invention are NPY Y5 receptor antagonists that effect NPY Y5 receptor mediated conditions. Therefore, they are expected to be valuable therapeutic agents useful in the treatment of NYP Y5 receptor mediated conditions such as obesity, bulimia nervosa, sexual/reproductive disorder, depression, anxiety, gastric ulcer, memory loss, migraine, pain, epileptic seizure, hypertension, cerebral hemorrhage, shock, congestive heart failure, sleep disturbance, nasal congestion, and diarrhea. An embodiment of this invention includes a method of treating NPY Y5 receptor mediated conditions in a mammal which comprises administering to said mammal a composition containing an amount of the compound of Formula I that is effective in treating the target condition.

Since the NPY Y5 receptor is linked to such abnormal conditions as obesity, bulimia nervosa, sexual/reproductive disorder, depression, anxiety, gastric ulcer, memory loss, migraine, pain, epileptic seizure, hypertension, cerebral hemorrhage, shock, congestive heart failure, sleep disturbance, nasal congestion, and diarrhea, an antagonist specific to the NPY Y5 receptor would act to inhibit these NPY Y5 mediated abnormal conditions and thereby have a beneficial pharmacological effect. The specificity of the compounds of this invention as NPY Y5 receptor antagonists can readily be determined by evaluating the affinity of the compound for the different NPY receptor subtypes and comparing the various receptor subtype affinities to discover specificity as well as activity. This can be determined by standard and well-known procedures.

The utility of the present invention as NPY Y5 receptor antagonists useful in treating NPY Y5 receptor mediated conditions can be demonstrated by the following procedures.

For instance, test compounds antagonistic to human NPY Y1 receptor subtype can be identified by a modified method of Gordon et al., *J. Neurochem.* **55**:506-513,(1990), wherein SK-N-MC cells (ATCC, Rockville, ND) are plated in 24-well plates. Once confluent, the cells are rinsed with Dulbecco's phosphate buffered saline (DPBS). Cells are then preincubated in binding buffer containing serum-free DMEM, 25 mM HEPES (pH 7.3), 0.5% bovine serum albumin (BSA), 0.1% bacitracin and 0.1mM phenylmethylsulfonyl fluoride for 30 minutes at room temperature. Test compounds at a final concentration of 5 µM, and [¹²⁵I] PYY (about 50 pM:NEN-DuPont) are added to the wells, and the cells are incubated for an additional 3 hours at room temperature, followed by rinsing with ice-cold DPBS. Nonspecific binding is defined with 1 µM NPY. After lysing the cells with 1% Triton X-100, the amount of radioactivity in the lysates is quantitated with a gamma counter. The results of compounds tested according to this protocol are reported in Table 2

Assays to determine binding affinity for human NPY Y2 and Y4/PP1 receptor subtypes are performed on GF/C Millipore 96-well plates pretreated with 0.02% polyethylenimine. The binding buffer for rat NPY Y2 receptor subtype binding is Krebs-Ringer bicarbonate (pH 7.4) containing 0.01% BSA and 0.005% bacitracin. Samples consist of membrane protein, 25 pM [¹²⁵I]PYY and test compounds at 5 µM final concentration. Nonspecific binding is defined by 1 µM NPY. The binding buffer for human Y4/PP1 binding consists of 137 mM NaCl, 5.4 mM KCl, 0.44 mM KH₂PO₄, 1.26 mM CaCl₂, 0.81 mM MgSO₄, 20 mM HEPES, 1 mM dithiothreitol, 0.1% bacitracin, 100 mg/l streptomycin sulfate, 1 mg/l aprotinin, 10 mg/ml soybean trypsin inhibitor and 0.3% BSA, pH 7.4. Samples consist of membrane protein, 50 pM human [¹²⁵I] human PP (hPP:NEN DuPont, Boston MA) and test compounds diluted to a final concentration of 5 µM. One µM hPP is used to define nonspecific binding. After a 2 hour incubation at room temperature with constant mixing, the samples are aspirated on a vacuum manifold, and rinsed with ice-cold binding buffer. The amount of radioactivity in each well is quantitated with either gamma counting or liquid scintillation. The results of compounds tested according to this protocol are reported in Table 2.

Assays to determine human and rat NPY Y5 receptor subtype binding affinity are performed on GF/C Millipore 96-well plates pretreated with 0.02% polyethylenimine. The binding buffer is 25 mM Tris, 120 mM NaCl, 5 mM KCl, 1.2 mM KH₂PO₄, 2.5 mM CaCl₂, 1.2 mM MgSO₄, 0.1% BSA and 0.5 mg/ml bacitracin, pH 7.4. Samples consist of membrane protein, 75-100 pM [¹²⁵I] PYY (porcine, NEN-DuPont) and test compounds diluted in binding buffer. Nonspecific binding is defined by 1 µM PYY. After a 2 hour incubation at room temperature with constant mixing, the samples are aspirated on a vacuum manifold and rinsed with ice-cold binding buffer. The amount of radioactivity in each well is quantitated with either gamma counting or liquid scintillation. IC₅₀ values, which correspond to 50% inhibition of specific binding, are determined with non-linear regression analysis. The results of compounds tested according to this protocol are reported in Table 2.

An *in vitro* functional assay to determine rat NPY Y5 receptor subtype binding affinity is performed by resuspending cells stably expressing the rat NPY Y5 receptor in serum-free DMEM containing 10 mM HEPES (pH 7.4) and 1 mM isobutylmethylxanthine (IBMX). One µM forskolin and test compounds at a final concentration of 10 µM are then added to the cells. After a 20 minute incubation of the samples at 37°C, the assay is stopped by placing the samples in boiling water for 3 minutes. The cAMP produced in each sample is quantitated with a radioimmunoassay kit (NEN DuPont). Data are expressed as a percentage of forskolin-stimulated adenylate cyclase. Data between 80 - 120% indicates no significant agonistic effect. The results of compounds tested according to this protocol are reported in Table 2.

In tests utilizing the above described procedures, the test compounds of the present invention were found to have selective NPY Y5 antagonist activity, as summarized in Table 2 below.

**Table 2**

| Compound No. | IC50 (µM) NPY receptor indicated* | | | | | % forskolin at 10 µM |
|---|---|---|---|---|---|---|
| | hY1 | hY2 | hY4/pp1 | hY5 | rY5 | |
| 1 | >5 | >5 | >5 | 10,000 | 9.8 | 78 |
| 2 | >5 | >5 | >5 | 0.83 | 0.46 | ND |
| 3 | ND | >5 | >5 | ND | 30%@1µM | ND |
| 4 | ND | >5 | >5 | ND | 2%@1µM | ND |
| 5 | ND | >5 | >5 | 0.025 | 0.025 | ND |
| 6 | >5 | >5 | >5 | 8.8 | 10 | 89 |
| 7 | >5 | 8.3 | >5 | 0.06 | 0.0526 | 122 |
| 8 | >5 | >5 | >5 | 0.022 | 0.011 | 105 |
| 9 | >5 | ND | >5 | 0.015 | 0.014 | 105 |
| 10 | >5 | >5 | >5 | 0.011 | 0.0067 | 106 |
| 11 | ND | >5 | >5 | 0.008 | ND | ND |
| 12 | >5 | >5 | >5 | 0.011 | ND | ND |
| 13 | ND | >5 | >5 | 0.045 | 0.036 | ND |
| 14 | >5 | >5 | >5 | 0.08 | ND | 99 |
| 15 | >5 | >5 | >5 | 0.005 | ND | ND |

| | | | | | | |
|---|---|---|---|---|---|---|
| *h means human; r means rat | | | | | | |

Based upon the above and other standard laboratory techniques known to evaluate compound receptor site inhibition, by standard toxicity tests and by standard pharmacological assays for the determination of treatment of the NPY Y5 mediated conditions identified above in mammals, and by comparison of these results with the results of known medicaments that are used to treat these conditions, the effective dosage of the compounds of this invention can readily be determined for treatment of each desired indication. The amount of the active ingredient to be administered in the treatment of one of these conditions can vary widely according to such considerations as the particular compound and dosage unit employed, the mode of administration, the period of treatment, the age and sex of the patient treated, and the nature and extent of the condition treated.

The total amount of the active ingredient to be administered will generally range from about 0.01 mg/kg to about 100 mg/kg, and preferably from about 0.1 mg/kg to about 20 mg/kg body weight per day. A unit dosage may contain from about 5 mg to about 1500 mg of active ingredient, and can be administered one or more times per day. Of course the specific initial and continuing dosage regimen for each patient will vary according to the nature and severity of the condition as determined by the attending diagnostician.

The compounds of this invention can be utilized to achieve the desired pharmacological effect by administration to a patient in need thereof in an appropriately formulated pharmaceutical composition. A patient, for the purpose of this invention, is a mammal, including a human, in need of treatment for a particular NPY Y5 receptor mediated condition, injury or disease. Therefore, the present invention includes pharmaceutical compositions which are comprised of a pharmaceutically acceptable carrier and a pharmaceutically effective amount of a compound of Formula I. A pharmaceutically acceptable carrier is any carrier which is relatively non-toxic and innocuous to a patient at concentrations consistent with effective activity of the active ingredient so that any side effects ascribable to the carrier do not vitiate the beneficial effects of the active ingredient. A pharmaceutically effective amount of compound is that amount which produces a result or exerts an influence on the particular condition being treated. The compounds of Formula I can be administered with a pharmaceutically-acceptable carrier using any effective conventional dosage unit forms, including immediate and timed release preparations, orally, parenterally, topically, or the like.

For oral administration, the compounds can be formulated into solid or liquid preparations such as capsules, pills, tablets, troches, lozenges, melts, powders, solutions, suspensions, or emulsions, and may be prepared according to methods known to the art for the manufacture of pharmaceutical compositions. The solid unit dosage forms can be a capsule which can be of the ordinary hard- of soft-shelled gelatin type containing, for example, surfactants, lubricants, and inert fillers such as lactose, sucrose, calcium phosphate, and corn starch.

In another embodiment, the compounds of this invention may be tableted with conventional tablet bases such as lactose, sucrose and cornstarch in combination with binders such as acacia, cornstarch or gelatin, disintegrating agents intended to assist the break-up and dissolution of the tablet following administration such as potato starch, alginic acid, corn starch, and guar gum, lubricants intended to improve the flow of tablet granulation and to prevent the adhesion of tablet material to the surfaces of the tablet dies and punches, for example talc, stearic acid, or magnesium, calcium or zinc stearate, dyes coloring agents, and flavoring agents intended to enhance the aesthetic qualities of the tablets and make them more acceptable to the patient. Suitable excipients for use in oral liquid dosage forms include diluents such as water and alcohols, for example, ethanol, benzyl alcohol, and polyethylene alcohols, either with or without the addition of a pharmaceutically acceptable surfactant, suspending agent or emulsifying agent.

Dispersible powders and granules are suitable for the preparation of an aqueous suspension. They provide the active ingredient in admixture with a dispersing or wetting agent, a suspending agent and one or more preservatives. Suitable dispersing or wetting agents and suspending agents are exemplified by those already mentioned above. Additional excipients, for example those sweetening, flavoring and coloring agents described above, may also be present.

The pharmaceutical compositions of this invention may also be in the form of oil-in-water emulsions. The oily phase may be a vegetable oil such as liquid paraffin or a mixture of vegetable oils. Suitable emulsifying agents may be (1) naturally occurring gums such as gum acacia and gum tragacanth, (2) naturally occurring phosphatides such as soy bean and lecithin, (3) esters or partial esters derived form fatty acids and hexitol anhydrides, for example, sorbitan monooleate, (4) condensation products of said partial esters with ethylene oxide, for example, polyoxyethylene sorbitan monooleate. The emulsions may also contain sweetening and flavoring agents.

Oily suspensions may be formulated by suspending the active ingredient in a vegetable oil such as, for example, arachis oil, olive oil, sesame oil or coconut oil, or in a mineral oil such as liquid paraffin. The oily suspensions may contain a thickening agent such as, for example, beeswax, hard paraffin, or cetyl alcohol. The suspensions may also contain one or more preservatives, for example, ethyl or n-propyl p-hydroxybenzoate; one or more coloring agents; one or more flavoring agents; and one or more sweetening agents such a sucrose or saccharin.

Syrups and elixirs may be formulated with sweetening agents such as, for example, slycerol, propylene glycol, sorbitol or sucrose. Such formulations may also contain a demulcent, and preservative and flavoring and coloring agents.

The compounds of this invention may also be administered parenterally, that is, subcutaneously, intravenously, intramuscularly, or interperitoneally, as injectable dosages of the compound in a physiologically acceptable diluent with a pharmaceutical carrier which can be a sterile liquid or mixture of liquids such as water, saline, aqueous dextrose and related sugar solutions, an alcohol such as ethanol, isopropanol, or hexadecyl alcohol, glycols such as propylene glycol or polyethylene glycol, glycerol ketals such as 2,2-dimethyl-1,1-dioxolane-4-methanol, ethers such as poly(ethyleneglycol) 400, an oil, a fatty acid, a fatty acid ester or glyceride, or an acetylated fatty acid glyceride with or without the addition of a pharmaceutically acceptable surfactant such as a soap or a detergent, suspending agent such as pectin, carbomers, methycellulose, hydroxypropylmethylcellulose, or carboxymethylcellulose, or emulsifying agent and other pharmaceutical adjuvants.

Illustrative of oils which can be used in the parenteral formulations of this invention are those of petroleum, animal, vegetable, or synthetic origin, for example, peanut oil, soybean oil, sesame oil, cottonseed oil, corn oil, olive oil, petrolatum and mineral oil. Suitable fatty acids include oleic acid, stearic acid, and isostearic acid. Suitable fatty acid esters are, for example, ethyl oleate and isopropyl myristate. Suitable soaps include fatty alkali metal, ammonium, and triethanolamine salts and suitable detergents include cationic detergents, for example dimethyl dialkyl ammonium halides, alkyl pyridinium halides, and alkylamine acetates; anionic detergents, for example, alkyl, aryl, and olefin sulfonates, alkyl, olefin, ether, and monoglyceride sulfates, and sulfosuccinates; nonionic detergents, for example, fatty amine oxides, fatty acid alkanolamides, and polyoxyethylenepolypropylene copolymers; and amphoteric detergents, for example, alkyl-beta-aminopropionates, and 2-alkylimidazoline quarternary ammonium salts, as well as mixtures.

The parenteral compositions of this invention will typically contain from about 0.5% to about 25% by weight of the active ingredient in solution. Preservatives and buffers may also be used advantageously. In order to minimize or eliminate irritation at the site of injection, such compositions may contain a non-ionic surfactant having a hydrophile-lipophile balance (HLB) of from about 12 to about 17. The quantity of surfactant in such formulation ranges from about 5% to about 15% by weight. The surfactant can be a single component having the above HLB or can be a mixture of two or more components having the desired HLB.

Illustrative of surfactants used in parenteral formulations are the class of polyethylene sorbitan fatty acid esters, for example, sorbitan monooleate and the high molecular weigth adducts fo ethylene oxide with a hydrophobic base, formed by the condensation of propylene oxide with propylene glycol.

The pharmaceutical compositions may be in the form of sterile injectable aqueous suspensions. Such suspensions may be formulated according to known methods using suitable dispersing or wetting agents and suspending agents such as, for example, sodium carboxymethylcellulose, methylcellulose, hydroxypropylmethyl-cellulose, sodium alginate, polyvinylpyrrolidone, gum tragacanth and gum acacia; dispersing or wetting agents which may be a naturally occurring phosphatide such as lecithin, a condensation product of an alkylene oxide with a fatty acit, for example, polyoxyethylene stearate, a condensation product of ethylene oxide with a long chain aliphatic alcohol;-for example, heptadecaethyleneoxycetanol, a condensation product of ethylene oxide with a partial ester derived form a fatty acid and a hexitol such as polyoxyethylene sorbitol monooleate, or a condensation product of an ethylene oxide with a partial ester derived from a fatty acid and a hexitol anhydride, for example polyoxyethylene sorbitan monooleate.

The sterile injectable preparation may also be a sterile injectable solution or suspension in a non-toxic parenterally acceptable diluent or solvent. Diluents and solvents that may be employed are, for example, water, Ringer's solution, and isotonic sodium chloride solution. In addition, sterile fixed oils are conventionally employed as solvents or suspending media. For this purpose, any bland, fixed oil may be employed including synthetic monoor diglycerides. In addition, fatty acids such as oleic acid can be used in the preparation of injectables.

A composition of the invention may also be administered in the form of suppositories for rectal administration of the drug. These compositions can be prepared by mixing the drug with a suitable non-irritation excipient which is solid at ordinary temperatures but liquid at the rectal temperature and will therefore melt in the rectum to release the drug. Such material are, for example, cocoa butter and polyethylene glycol.

The compositions of the invention can also contain other conventional pharmaceutically acceptable compounding ingredients, generally referred to as carriers or diluents, as necessary or desired. Any of the compositions of this invention may be preserved by the addition of an antioxidant such as ascorbic acid or by other suitable preservatives. Conventional procedures for preparing such compositions in appropriate dosage forms can be utilized.

The compounds of this invention can be administered as the sole pharmaceutical agent or in combination with one or more other pharmaceutical agents where the combination causes no unacceptable adverse effects. For example, the compounds of this invention can be combined with known anti-obesity or other indication agents, and the like, as well as with admixtures and combinations thereof.

Typically, the individual daily dosages for these combinations can range from about one-fifth of the minimally recommended clinical dosages to the maximum recommended levels for the agents when they are administered singly.

The following specific examples are presented to illustrate the inventions described herein, but they should not be construed as limiting the scope of these inventions in any way.

All starting materials, reagents and solvents in the following specific examples were obtained from commercial suppliers and, unless otherwise noted, were used without further purification.

The following solvent systems were used for analytical thin-layer chromatography (TLC): (A) 80:20 hexane : ethyl acetate, (B) 95:5 toluene : ethyl acetate, (C) 50:50 hexane : ethyl acetate. TLC was performed on Merck Kieselgel 60 F₂₅₄ silica gel plates. Detection was effected by exposure to UV light (254 nm) or by immersion in basic aqueous potassium permanganate solution. Chromatography was performed using Silica Gel 60 (#9385-5) from EM Science. Melting points were recorded in open capillary tubes and are uncorrected. ¹H NMR spectra were determined at 300 MHz using a General Electric GE-OMEGA 300 spectrometer. Chemical shifts are reported in parts per million (δ) values relative to tetramethylsilane as internal standard. Spin multiplicities are reported using the following abbreviations: singlet (s), doublet (d), doublet of doublets (dd), triplet (t), quartet (q), multiplet (m), and broad (br). Coupling constants are in Hertz. Fast atom bombardment (FAB) mass spectra were recorded using a Kratos Concept 1 spectrometer; electron impact (EI) and chemical ionization (CI) mass spectra were recorded using a Hewlett-Packard MS Engine (HP5989A) spectrometer; liquid chromatography-mass spectra (LC-MS) were recorded using a Finningan MAT LCQ spectrometer.

### EXAMPLE 1

### Novel Intermediate No. 1

### 2-Phenyl-1-(quinolin-2-yl)ethanol

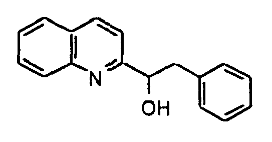

2-Quinolinecarboxaldehyde (5.0 g, 31.8 mM) was dissolved in anhydrous THF (50 mL) at room temperature with stirring under an argon atmosphere and the resulting solution was cooled to -78°C in a dry-ice bath. To this was added benzylmagnesium chloride (47.7 mL, 47.7 mmol, 1 M in Et₂O) over a 10 minute period via syringe. The mixture was allowed to stir at this temperature for 10 minutes then removed from the bath. Once the reaction mixture reached room temperature saturated NH₄Cl was added and the product extracted with ethyl acetate. The organic layer was washed with water followed by a saturated brine solution, dried (MgSO₄), filtered and concentrated *in vacuo*. The residue was triturated with hexanes to provide 5.59 g (70%) of the title compound as a brown powder: ¹H NMR (300 MHz, DMSO-d₆) δ 2.95 (dd, J = 13.60 Hz, 8.46 Hz, 1 H), 3.15 (dd, J = 13.60, 4.78 Hz, 1 H), 4.95 (m, 1 H), 5.60 (d, J = 5.15 Hz, 1 H), 7.09-7.29 (m, 5 H), 7.54 (m, 1 H), 7.62 (d, J = 9.0 Hz, 1 H), 7.72 (m, 1 H), 7.93 (t, J = 9.0 Hz, 2 H), 8.30 (d, J = 9.0 Hz, 1 H).

### EXAMPLE 2

### Intermediate No. 2

### 2-Phenyl-1-(quinotin-2-yl)-1-oxoethane

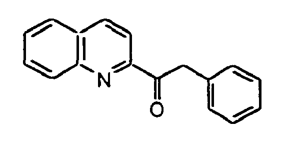

The compound of Example 1 (2.72 g, 10.9 mmol) and triethylamine (9.13 mL, 65.5 mmol) were dissolved in anhydrous DMSO (100 mL) at room temperature with stirring. Sulfur trioxide pyridine complex (5.21 g, 32.8 mmol) was added to the mixture portionwise to prevent a rise in temperature. The mixture was then allowed to stir at room temperature for 2.5 hr and then poured into water. The product was extracted into ethyl acetate, washed with water followed by a saturated brine solution. The organic layer was dried (MgSO₄), filtered and concentrated *in vacuo.* The residue was purified by column chromatography (8:1 hexane : ethyl acetate) to provide 1.42 grams (53%) of the title compound as a brown powder.

### EXAMPLE 3

### Compound No. 1

### (4-Methylphenylsulfonyl)-1-oxo-2-phenyl-1-(quinolin-2-yl)propane

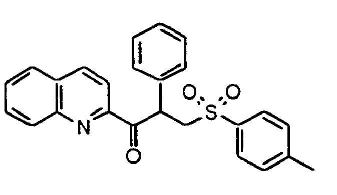

The following procedure was adapted from a procedure described by Hellmann and Mueller (*Chem. Ber.* 1965, 98, 638) The compound of Example 2 (357 mg, 1.40 mmol), paraformaldehyde (54.2 mg, 1.80 mmol), and sodium p-toluenesulfinate x 2 H₂O ( 464.5 mg, 2.20 mmol) were dissolved in 15 mL of DMF at room temperature. Acetic acid (130 mg, 2.2 mmol) was added to the mixture via syringe. The mixture was warmed at 45°C for 2 hrs, then at 90°C for an additional 2 hr. The reaction was cooled to room temperature and poured into water. The product was extracted into ethyl acetate. The organic layer was washed with water followed by a saturated brine solution, dried (MgSO₄), filtered and concentrated *in vacuo.* The residue was purified by flash chromatography (2:1 hexanes : ethyl acetate) to provide 350 mg (58%) of a yellow solid. This material was recrystallized to give 200 mg of the title compound as a cream colored solid: MP = 140-144°C; ¹H NMR (300 MHz, DMSO-d₆) δ 2.36 (s, 3 H), 3.85 (dd, J = 14.34 Hz, 4.05 Hz, 1 H), 4.40 (dd, J = 14.0 Hz, 10.0 Hz, 1 H), 6.09 (dd, J = 9.93 Hz, 3.68 Hz, 1 H), 7.09-7.21 (m, 3 H), 7.29-7.36 (m, 4 H), 7.71 (d, J = 8.46 Hz, 2 H), 7.78 (m, 1 H), 7.91-7.97 (m, 2 H), 8.08 (d, J = 8.46 Hz, 1 H), 8.26 (d, J = 8.09 Hz, 1 H), 8.51 (d, J = 8.46 Hz, 1 H); MS (FAB) m/z 416 (M + H)⁺.

### EXAMPLE 4

### Intermediate No. 3

### N-Methoxy-N-methyl-2-phenyl-acetamide

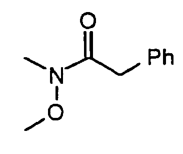

Phenyl acetic acid (10.00 g, 73.5 mmol) and N,O-dimethylhydroxylamine hydrochloride (7.15, 73.5 mmol) was dissolved in CH₂Cl₂ (150 mL) with stirring under an argon atmosphere. The solution was cooled to 0°C in an ice bath. To this solution was added 1-(3-dimethylaminopropyl)-3-ethylcarbodiimide hydrochloride (EDCI, 17.60 g, 92.0 mmol) and the reaction was allowed to warm to room temperature over 1 h. The mixture was poured into a separatory funnel that contained 10% HCl (50 mL). The solution was extracted with ethyl acetate. The organic layer was dried ( MgSO₄), filtered, and concentrated *in vacuo.* The residue was purified by flash chromatography (3: 1 hexane : ethyl acetate) to provide 12.5 g of the title compound as a clear oil: ¹H NMR (300 MHz, CDCl₃) δ 3.20 (s, 2 H), 3.61 (s, 3 H), 3.79 (s, 3 H), 7.31 (m, 5 H).

### EXAMPLE 5

### Novel Intermediate No. 4

### 2-Phenyl-1-[1-(2-trimethylsilanyl-ethoxymethyl)-1H-imidazol-2-yl]-ethanone

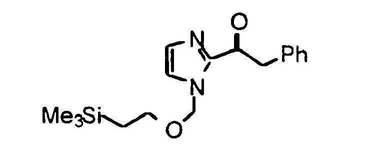

(2-trimethylsilanyl-ethoxymethyl)-1*H*-imidazole (0.5 g, 2.46 mmol; *J. Org. Chem*. **1986**, 51, 1891-1894.) was dissolved in anhydrous THF (20 mL) at room temperature with stirring under an argon atmosphere. The solution was cooled to -78°C in a dry-ice/acetone bath. To this solution was added n-butyl lithium (2 M solution, 1.6 mL, 2.59 mmol). The mixture was allowed to stir at -78°C for one hour. The compound of Example 4 (0.44 g, 2.46 mmol) was then added and stirring was continued at -78°C for one hour. The mixture was concentrated *in vacuo* and purified by flash chromatography to provide 0.360 g of the title compound as a clear oil: ¹H NMR (300 MHz, CDCl₃) δ 0.00 (s, 9 H), 0.92 (dd, J = 8.1 Hz, 8.1 Hz, 2 H), 3.55 (dd, J = 8.1 Hz, 8.1 Hz, 2 H), 4.49 (s, 2 H), 5.76 (s, 2 H), 7.32 (m, 7 H); Rf (3:1 hexane : ethyl acetate) = 0.45.

### EXAMPLE 6

### Novel Intermediate No. 5

### 2-Phenyl-3-p-tolylsulfanyl-1-[1-(2-trimethylsilanyl-ethoxymethyl)-1H-imidazol-2-yl]-propan-1-one

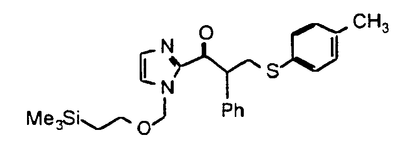

The following was adapted from the literature procedure described by Schaller (DE 3527334 A1): *p*-Thiocresol (0.039 g, 0.31 mmol), paraformaldehyde (0.009 g, 0.31 mmol), piperidine (0.02 mL, 0.20 mmol), and acetic acid (0.008 g, 0.13 mmol) were mixed together in toluene (15 mL) at room temperature with stirring under an argon atmosphere. The compound of Example 5 (0.100g, 0.31 mmol) was added and the mixture was heated to reflux for 16 h, with a Dean-Stark trap. The mixture was concentrated *in vacuo* and the residue was dissolved in ethyl acetate. The ethyl acetate solution was washed with water, saturated brine solution, dried (MgSO₄), filtered and concentrated *in vacuo.* The residue was purified by flash chromatography (25% ethyl acetate in hexanes) to provide 0.12 g of the title compound as a white solid: ¹H NMR (300 MHz, CDCl₃) δ 0.00 (s, 9 H), 0.92 (m, 2 H), 2.37 (s, 3 H), 3.32 (dd, J = 12.9 Hz, 5.5 Hz, 1 H), 3.53 (m, 2 H), 3.82 (dd, J = 13.1 Hz, 9.5 Hz, 1 H), 5.52 (dd, J = 9.8 Hz, 5.1 Hz, 1 H), 5.80 (q; J = 15.8 Hz, 10.3 Hz, 2 H), 7.31 (m, 11 H); Rf (3:1 hexanes : ethyl acetate) = 0.48.

### EXAMPLE 7

### Compound No. 2

### (1H-lmidazol-2-yl)-2-phenyl-3-p-tolylsulfanyl-propan-1-one

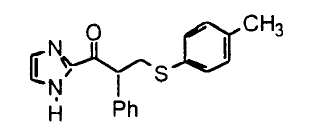

The compound of Example 12 (130 mg, 0.29 mmol) was dissolved in ethanol (6 mL) at room temperature with stirring under an argon atmosphere. To this was added conc. HCl (6 mL) and the mixture was heated to 65°C for 2.5 h. 20% NaOH was added to the mixture until pH=14, then extracted with ethyl acetate. The organic layer was dried (MgSO₄), filtered, and concentrated *in vacuo* to provide 0.10 g of the title compound as a white solid: ¹H NMR (300 MHz, CDCl₃) δ 2.28 (s, 3 H), 3.25 (dd, J = 13.1 Hz, 5.5 Hz, 1 H), 3.75 (dd, J = 13.1 Hz, 9.6 Hz, 1 H), 5.27 (dd, J = 9.8 Hz, 5.5 Hz, 1 H), 7.21 (m, 11 H), 10.70 (s, 1 H).

### EXAMPLE 8

### Compound No. 3

### (1H-lmidazol-2-yl)-2-phenyl-3-(toluene-4-sulfonyl)-propan-1-one

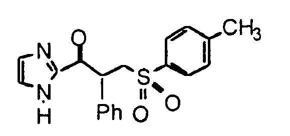

The compound of Example 4 (0.05 g, 0.16 mM) was dissolved in CH₂Cl₂ (15 mL) at room temperature with stirring under an argon atmosphere. The solution was cooled to 0°C in an ice bath. To this was added a quantity of 3-Chloroperoxybenzoic acid (57-86%, 0.064 g). The mixture was allowed to warm to room temperature and stirred for 3.5 h. Sodium hydrogen sulfite was added and the mixture was extracted with CHCl₃. The organic layer was dried (MgSO₄), filtered, and concentrated *in vacuo.* The residue was purified by flash chromatography (5% methanol/CH₂Cl₂) to provide 0.03 g of the title compound as a white solid: ¹H NMR (300 MHz, CDCl₃) δ 2.34 (s, 3 H), 3.37 (dd, J = 14.3 Hz, 2.6 Hz, 1 H), 4.39 (dd, J = 14.3 Hz, 10.7 Hz, 1 H), 5.50 (dd, J = 10.8 Hz, 2.7 Hz, 1 H), 7.18 (m, 11 H), 7.69 (d, J = 8.45 Hz, 1 H); Rf (5% methanol/CH₂Cl₂) = 0.25.

### EXAMPLE 9

### Novel Intermediate No. 6

### 2-Phenyl-1-[1-(2-trimethylsilanyl-ethoxymethyl)-1H-benzoimidazol-2-yl]-ethanone

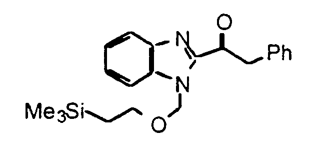

(2-trimethylsilanyl-ethoxymethyl)-1*H*-benzoimidazole (5.5 g, 22.1 mmol; *J. Org. Chem.* **1986**, *51*, 1891-1894.) was suspended in anhydrous THF (100 mL) and cooled to -78°C under an argon atmosphere. 2.5 M n-butyl lithium (8.9 mL, 22.1 mmol) was added over a 1 minute period and the reaction was allowed to stir at -78°C for 1 h. A solution of the compound of Example 4 (4.0 g, 22.1 mmol) in THF (10 mL) was added, and the reaction was stirred an additional 2 h at -78°C. The reaction was quenched and then extracted with ether (3 X 20 mL). The solvent was removed *in vacuo* and the resulting oil was purified by flash chromatography to provide 3.6 g of the title compound as an oil: ¹H NMR (300 MHz, CDCl₃) δ 0.00 (s, 9 H), 0.95 (dd, 2 H), 3.59 (dd, 2 H), 4.79 (s, 2 H), 6.17 (s, 2 H), 7.41-7.63 (m, 7 H), 7.76 (d, 1 H), 8.08 (d, 1 H).

### EXAMPLE 10

### Novel Intermediate No. 7

### 2-Phenyl-3-p-tolylsulfanyl-1-[1-(2-trimethylsilanyl-ethoxymethyl)-1H-benzoimidazol-2-yl] -propan-1-one

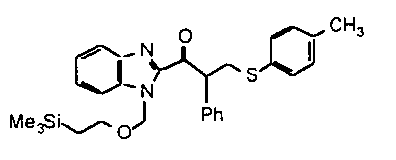

The following was adapted from the literature procedure described by Schaller (DE 3527334 A1): The compound of Example 9 (500 mg, 1.36 mmol) was suspended in toluene (5 mL) with p-thiocresol (169 mg, 1.36 mmol), paraformaldehyde (41 mg, 1.36 mmol), acetic acid (0.03 mL, 0.5 mmol), and piperidine (0.03 mL). The solution was refluxed under argon for 18 h in a set-up equipped with a Dean-Stark trap. After cooling, the solvent was removed under reduced pressure and the resulting oil was purified by flash chromatography (10% ethyl acetate in hexane) to provide 450 mg (66%) of the title compound: ¹H NMR (300 MHz, CDCl₃) δ 0.00 (s, 9 H), 0.89-0.96 (m, 2 H), 2.46 (s, 3 H), 3.43-3.58 (m, 3 H), 3.94 (dd, J = 12.9 Hz, 9.6 Hz, 1 H), 5.87 (dd, J = 9.9 Hz, 5.5 Hz, 1 H), 6.15 (q, J = 10.7 Hz, 2 H), 7.20-7.72 (m, 12 H), 8.06 (d, J = 8.5 Hz, 1 H).

### EXAMPLE 11

### Compound No. 4

### (1H-Benzoimidazol-2-yl)-2-phenyl-3-p-tolylsulfanyl-propan-1-one

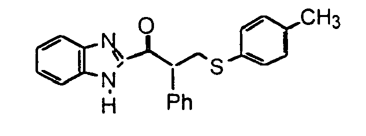

The compound of Example 10 (400 mg, 0.8 mmol) was suspended in ethanol (8 mL) and 3 N HCl (0.5 mL). After heating for 12 h at 50°C under argon, the reaction was cooled and most of the ethanol was removed under reduced pressure. Water (15 mL) was added, the product was extracted with CH₂Cl₂ (3 X 10 mL), and the organic layer was concentrated *in vacuo.* The residue was purified by flash chromatography to provide 170 mg of an oil which was titrated with hexane to give 83 mg of the title compound as a white powder: ¹H NMR (300 MHz, CDCl₃) d 2.27 (s, 3 H), 3.31 (dd, J = 13.1 Hz, 5.5, Hz, 1 H), 3.80 (dd, 1 H, J = 13.2 Hz, 9.9 Hz, 1 H), 5.50 (dd, J = 9.8 Hz, 5.2 Hz, 1 H), 7.05-7.69 (m, 13 H); MS (FAB) m/z 373 (M + H)⁺.

### EXAMPLE 12

### Compound No. 5

### (1H-Benzoimidazol-2-yl)-2-phenyl-3-(toluene-4-sulfinyl)-propan-1-one

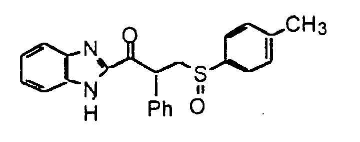

The compound of Example 11 (1.4 g, 3.8 mmol) was dissolved in CH₂Cl₂ (40 mL) and cooled in an ice bath. A quantity of 3-Chloroperoxybenzoic acid (57-86%, 1.15 g) was added and the solution was allowed to stir at 0°C for 45 min. before the addition of water (30 mL). The product was extracted with CH₂Cl₂ (3X), dried (Na₂SO₄), filtered and concentrated *in vacuo* to a residue. The residue was purified by flash chromatography (10% methanol in CH₂Cl₂) to provide 493 mg of the title compound as a mixture of diastereomers: ¹H NMR (300 MHz, CDCl₃) δ 2.33 and 2.41 (2 s, 3 H), 3.16 and 3.32 (2 dd, 1 H, J = 13.1 Hz, 2.9 Hz and 13.1 Hz, 5.1 Hz), 4.00 and 4.31 (dd, J = 13.2 Hz, 8.8 Hz and t, J = 12.9 Hz, 1 H), 5.69 and 5.92 (2 dd, J = 11.2 Hz, 3.0 Hz and 8.3 Hz, 4.8 Hz, 1 H), 7.15-8.0 (m, 13 H); Anal. calculated for C₂₃H₂₀N₂SO₂: C, 71.10; H, 5.19; N, 7.21; S, 8.25; Found: C, 70.95; H, 5.08; N, 7.16; S, 8.17.

### EXAMPLE 13

### Novel Intermediate No. 8

### 1-Benzothiazol-2-yl-2-phenyl-ethanone

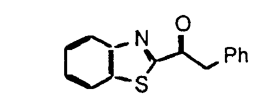

Benzothiazole (1.00 g, 7.40 mmol) was dissolved in anhydrous THF (30 mL) and cooled to -78°C under an argon atmosphere. 2.5 M n-butyl lithium (3 mL, 7.4 mmol, 1 eq) was added dropwise and the reaction was allowed to stir at -78°C for 20 min. A solution of the compound of Example 7 (1.33 g, 7.40 mmol, 1 eq) in THF (5 mL) was added dropwise, and the reaction was stirred an additional 3 h at -78°C. The reaction was quenched with a saturated solution of ammonium chloride (10 mL) and was then allowed to warm to room temperature. The resultant mixture was extracted with ethyl acetate (3 X 20 mL) and the combined organic layer was washed with a saturated solution of sodium chloride (20 mL), dried (Na₂SO₄), and absorbed on Celite through concentration under reduced pressure in the presence of Celite. The celite was loaded onto a column of silica, and the adsorbed mixture was purified by flash chromatography (5% ethyl acetate-hexane) to provide 800 mg (43%) of title compound as a near-white solid: ¹H NMR (300 MHz, CDCl₃) δ 4.60 (s, 2 H), 7.25-7.65 (m, 7 H), 7.99 (dd, J=8.4 Hz, 1.2 Hz, 1 H), 8.25 (dd, J=7.2 Hz, 1.2 Hz, 1 H); MS m/z 253 M⁺.

### EXAMPLE 14

### Compound No. 6

### 1-Benzothizol-2-yl-2-phenyl-3-(toluene-4-sulfonyl)-propan-1-one

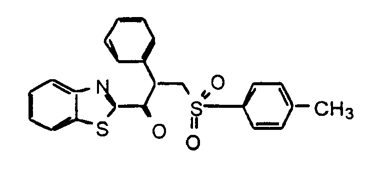

Prepared in 98% yield from the compound described in Example 13 using the procedure described in Example 3: MP = 135-137°C; ¹H NMR (300 MHz, DMSO-d₆) δ 2.34 (s, 3 H), 3.95 (dd, J = 14.34 Hz, 4.42 Hz, 1 H), 4.41 (dd, J = 14.34 Hz, 9.56 Hz, 1 H), 5.58 (dd, J = 9.56 Hz, 4.05 Hz, 1 H), 7.17-7.30 (m, 7H), 7.61-7.71 (m, 4 H), 8.23 (m, 1 H), 8.33 (m, 1 H); MS (FAB) m/z 422 (M + H)⁺; Anal. calculated for C₂₃H₁₉NO₃S₂: C, 65.53; H, 4.54; N, 3.32; Found: C, 65.30; H, 4.43; N, 3.31.

### EXAMPLE 15

### Intermediate No. 9

### 2-Hydroxy-3-Dhenyl-4-(toluene-4-sulfonyl)-cyclobut-2-enone

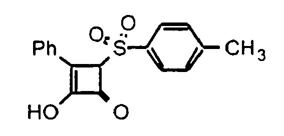

The following procedure is a modification of that reported by Reid et. al.(*Chem. Ber.* **1975,** 108, 538). Phenylcyclobutenedione (5.0 g, 31.6 mmol), *para-*toluenesulfinic acid (5.4 g, 34.6 mmol), and 4-dimethylaminopyridine ("DMAP", 0.39 g, 3.2 mmol) were dissolved in anhydrous THF (70 mL). The solution was refluxed for 2 hours, cooled to room temperature, and concentrated by rotary evaporation. The crude residue was titrated in chloroform to yield 7.80 g (79%) of the title compound as a yellow solid: ¹H NMR (300 MHz, acetone-d₆) δ 2.43 (s, 3 H), 5.27 (s, 1 H), 7.47 (d, 2 H), 7.50 (m, 3 H), 7.70 (d, 2 H), 7.89 (d, 2 H), 10.95 (bs, 1 H); MS (FAB) m/z 315 (M + H⁺), m/z 157 (-C₁₀H₆O₂); Rf (ethyl acetate) = 0.1; MP = 156-158°C

### EXAMPLE 16

### Compound No. 7

### (5,6-Dimethyl-1H-benzoimidazol-2-yl)-2-phenyl-3-(toluene-4-sulfonyl)-propan-1-one

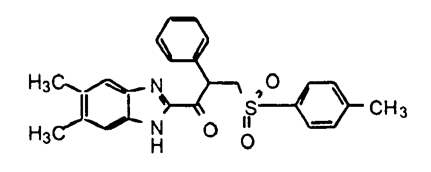

4,5-Dimethyl-1,2-phenylenediamine (0.55 g, 4.04 mmol) was dissolved in 10 mL of glacial acetic acid and added to the compound of Example 16 (1.1 g, 3.5 mmol) suspended in 20 mL of glacial acetic acid. The solution was stirred for 2 hours at room temperature. Acetic acid was removed *in vacuo* and the residue was purified by flash chromatography (5% ethyl acetate in toluene) to provide an orange solid. The solid was recrystallized from 2:1 toluene/hexanes to yield 0.42 g (82%) of the title compound as a white solid: ¹H NMR (300 MHz, CDCl₃) δ 2.37 (s, 9 H), 3.52 (d, 1 H), 4.52 (dd, 1 H), 5.78 (d, 1 H), 7.21 (m, 7 H), 7.83 (d, 2 H), 9.85 (bs, 1 H); MS (FAB) m/z 433 (M + H⁺) m/z 277 (M⁺-SO₂PhCH₃); MP = 173-176_C; Rf 0.2 (5% ethyl acetate in toluene).

In like manner, the following intermediate compounds can be prepared by substituting the appropriate starting materials to result in the appropriate moiety instead of the (3-methyl)phenyl moiety and phenyl moieties of the intermediate of Example 16, and following the process of Example 16 to make the following intermediate compounds:
Intermediate No. 10: 4-Benzenesulfonyl-2-hydroxy-3-phenyl-cyclobut-2-enone; MP 142-144°C; Ms 301 (M+H).
Novel Intermediate No. 11 3-(4-Fluoro-phenyl)-2-hydroxy-4-(toluene-4-sulfonyl)-cyclobut-2-enone; MP 162-164°C; Rf 0.1 (A).
Novel Intermediate No. 12: 2-Hydroxy-4-(4-methoxy-benzenesulfonyl)-3-phenyl-cyclobut-2-enone; MP 140-143°C; MS 331 (M+H).
Novel Intermediate No. 13: 4-(4-Bromo-benzenesulfonyl)-2-hydroxy-3-phenyl-cyclobut-2-enone.
Novel Intermediate No. 14 2-Hydoxy-4-(naphthalene-2-sulfonyl)-3-phenyl-cyclobut-2-enone; MP 129-132°C; MS 351 (M+H).

The above intermediates can in turn be used according to the process of Example 17 to make the corresponding following compounds:
Compound No. 8: 3-Benzenesulfonyl-1-(1*H*-benzoimidazol-2-yl)-2-phenyl-propan-1-one; MP 201-203°C; Rf 0.46(C); MS (LSIMS) 391 (M+H).
Compound No. 9: 1-(1*H*-Benzoimidazol-2-yl)-2-(4-fluoro-phenyl)-3-(toluene-4-sulfonyl)-propan-1-one; MP 191-193°C; Rf 0.29(B); 423(M+H)⁺, 267 (M-SO₂Tol)
Compound No. 10: 1-(1*H*-Benzoimidazol-2-yl)-3-(4-methoxy-benzenesulfonyl)-2-phenyl-propan-1-one; MP 209-211°C; Rf 0.05(C); 421 (M+H).
Compound No. 11: 1-(1*H*-Benzoimidazol-2-yl)-3-(4-bromo-benzenesulfonyl)-2-phenyl-propan-1-one; MP 208-210°C; 0.55(C); 471(M+H)⁺, 469(M+H).
Compound No. 12: 1-(1*H*-Benzoimidazol-2-yl)-3-(naphthalene-1-sulfonyl)-2-phenyl-propan-1-one; MP 216-218°C; 0.5(C); 441 (M+H).
Compound No. 13: 1-(5-Methyll-1*H*-benzoimidazol-2-yl)-2-phenyl-3-(toluene-4-sulfonyl)-propan-1-one; MP 159-161°C; 0.36(C); 419 (M+H).
Compound No. 14: 1-(5-Methoxy-1*H*-benzoimidazol-2-yl)-2-phenyl-3-(toluene-4-sulfonyl)-propan-1-one; MP 148-152°C; 0.36 (B); 435 (M+H).
Compound No. 15: 1-(1H-Benzoimidazol-2-yl)-2-phenyl-3-(toluene-4-sulfonyl)-propan-1-one; MP 218-220°C; 0.16(B); 405 (M+H).

### EXAMPLE 18

A tablet is prepared from

| | |
|---|---|
| 3-Benzenesulfonyl-1-(1*H*-benzoimidazol-2-yl) -2-phenyl-propan-1-one | 250 mg |
| Starch | 40 mg |
| Talc | 10 mg |
| Magnesium | 10 mg |

### EXAMPLE 19

A capsule is prepared from

| | |
|---|---|
| (5-Methyl-1*H*-benzoimidazol-2-yl)-2-phenyl (toluene-4-sulfonyl)-propan-1-one | 200 mg |
| Sodium carboxymethyl cellulose | 40 mg |
| Starch | 120 mg |

The compounds of Formula I may also be utilized, in free base form or in compositions, in research and diagnostics, or as analytical references or standards, and the like, according to methods well known in the art.

Therefore, the present invention includes compositions which are comprised of an inert carrier and an effective amount of a compound of Formula I. An inert carrier is any material which does not interact with the compound to be carried and which lends support, means of conveyance, bulk, traceable material, and the like to the compound to be carried. An effective amount of compound is that amount which produces a result or exerts an influence on the particular procedure being performed, and will be readily apparent to anyone skilled in the art.

It should be apparent to one of ordinary skill in the art that changes and modifications can be made to this invention without departing from the spirit or scope of the invention as it is set forth herein.

## Claims

1. A compound of the formula (I) 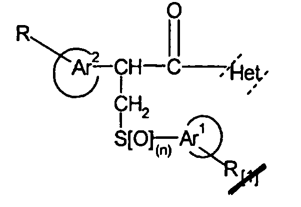 wherein
R is C₁-C₆-alkyl, C₁-C₆-alkoxy, F, Br, Cl and I;
Ar¹ is phenyl, naphthyl or thiophene, which can optionally be substituted
with R;
Ar² is phenyl or pyridyl, which can optionally be substituted with R;
n is 0, 1 or 2;
Het is quinoline, benzimidazole, benzthiazole, indole, indoline, purine, imidazole, pyrroline, pyrrolidine, pyrazole, pyrazoline or triazole, where the heteroatom groups of said moiety are in the α position of the carbon atom bound to the carbonyl atom, and where in one of the position alpha of the carbon bound to the carbonyl there is N atom
which can optionally be substituted with zero to six substituents as defined by R;
and pharmaceutically acceptable salts thereof, with the exception of the following specific compounds: 1-(2-benzothiazolyl)-3-[(4-methylphenyl) sulfonyl]-2-phenyl-1-propanone, 1-(1-methyl-1H-benzimidazole-2-yl)-3-[(4-methylphenyl)sulfonyl]-2-phenyl-1-propanone, 1-(1-H-benzimidazole-2-yl)-3-(4-[(methylphenyl)sulfonyl]-2phenyl-1-propanone and 1-(1H-benzimidazole-2-yl)-3-[phenyl(sulfonyl)]-2phenyl-1 propanone.

2. A compound of claim 1, wherein n is 2.

3. A compound of claim 1 or 2, wherein Ar¹ and Ar² are each phenyl optionally substituted with R.

4. A compound one of claims 1 to 3, wherein Het is benzimidazole optionally substituted with R.

5. A pharmaceutical composition comprising an effective amount of a compound of the formula (1) as defined in claims 1 to 4.

6. A composition comprising an effective amount of a compound of the formula (1) as defined in claims 1 to 4.

7. Use of a compound of the formula (1) as defined in claims 1 to 4 for the preparation of a pharmaceutical.

## Patentansprüche

1. Verbindung der Formel (I) 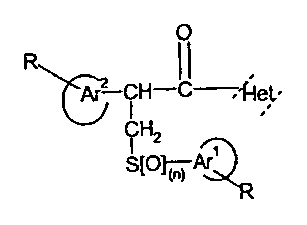 worin
R C₁-C₆-Alkyl, C₁-C₆-Alkoxy, F, Br, Cl oder I ist;
Ar¹ Phenyl, Naphthyl oder Thiophen ist, das gegebenenfalls mit R substituiert ist;
Ar² Phenyl oder Pyridyl ist, das gegebenenfalls mit R substituiert ist;
n = 0, 1 oder 2 ist;
Het Chinolin, Benzimidazol, Benzthiazol, Indol, Indolin, Purin, Imidazol, Pyrrolin, Pyrrolidin, Pyrazol, Pyrazolin oder Triazol ist, worin sich die Heteroatomgruppen dieser Gruppierung an der α-Position zum an das Carbonylatom gebundenen Kohlenstoffatom befinden und worin an einer α-Position zum an das Carbonyl gebundenen Kohlenstoffatom ein N-Atom vorhanden ist,
das gegebenenfalls mit null bis sechs Substituenten, wie sie für R definiert sind, substituiert sein kann;
und pharmazeutisch annehmbare Salze davon, mit Ausnahme der folgenden spezifischen Verbindungen: 1-(2-Benzothiazolyl)-3-[(4-methylphenyl)sulfonyl]-2-phenyl-1-propanon, 1-(1-Methyl-1H-benzimidazol-2-yl)-3-[(4-methylphenyl)sulfonyl]-2-phenyl-1-propanon, 1-(1-H-Benzimidazol-2-yl)-3-(4-[(methylphenyl)sulfonyl]-2-phenyl-1-propanon und 1-(1H-Benzimidazol-2-yl)-3-[phenyl(sulfonyl)]-2-phenyl-1-propanon.

2. Verbindung nach Anspruch 1, worin n = 2 ist.

3. Verbindung nach Anspruch 1 oder 2, worin Ar¹ und Ar² jeweils Phenyl sind, das gegebenenfalls mit R substituiert ist.

4. Verbindung nach einem der Ansprüche 1 bis 3, worin Het Benzimidazol ist, dass gegebenenfalls mit R substituiert ist.

5. Pharmazeutische Zusammensetzung, eine wirksame Menge einer Verbindung der Formel (1) nach einem der Ansprüche 1 bis 4 umfassend.

6. Zusammensetzung, eine wirksame Menge einer Verbindung der Formel (1) nach einem der Ansprüche 1 bis 4 umfassend.

7. Verwendung einer Verbindung der Formel (1) nach einem der Ansprüche 1 bis 4 zur Herstellung eines Pharmazeutikums.

## Revendications

1. Composé de formule (I) 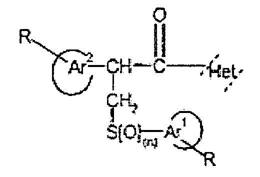 dans laquelle
R représente un reste alkyle en C₁ à C₆, alkoxy en C₁ à C₆, F, Br, Cl et I ;
Ar¹ est un reste phényle, naphtyle ou thiophène, qui peut facultativement être substitué avec R ;
Ar² est un reste phényle ou pyridyle, qui peut facultativement être substitué avec R ;
n a la valeur 0, 1 ou 2 ;
Het est un groupement quinoléine, benzimidazole, benzothiazole, indole, indoline, purine, imidazole, pyrroline, pyrrolidine, pyrazole, pyrazoline ou triazole, les groupes à hétéroatome de ce groupement se trouvant dans la position α de l'atome de carbone lié à l'atome du carbonyle, et dans l'une des positions alpha de l'atome de carbone lié au carbonyle se trouve un atome d'azote
qui peut facultativement être substitué par zéro à six substituants tels que définis par R ;
et ses sels pharmaceutiquement acceptables, à l'exception des composés suivants : 1-(2-benzothiazolyl)-3-[(4-méthylphényl)sulfonyl]-2-phényl-1-propanone, 1-(1-méthyl-1H-benzimidazole-2-yl)-3-[(4-méthylphényl)sulfonyl]-2-phényl-1-propanone, 1-(1-H-benzimidazole-2-yl)-3-(4-[(méthylphényl)-sulfonyl]-2-phényl-1-propanone et 1-(1H-benzimidazole-2-yl)-3-[phényl(sulfonyl)]-2-phényl-1-propanone.

2. Composé suivant la revendication 1, dans lequel n est égal à 2.

3. Composé suivant la revendication 1 ou 2, dans lequel Ar¹ et Ar² représentent chacun un reste phényle facultativement substitué avec R.

4. Composé suivant l'une des revendications 1 à 3, dans lequel Het est un groupement benzimidazole facultativement substitué avec R.

5. Composition pharmaceutique, comprenant une quantité efficace d'un composé répondant à la formule (I) telle que définie dans les revendications 1 à 4.

6. Composition, comprenant une quantité efficace d'un composé répondant à la formule (I) telle que définie dans les revendications 1 à 4.

7. Utilisation d'un composé de formule (I) telle que définie dans les revendications 1 à 4 pour la préparation d'un produit pharmaceutique.
